# EUROPEAN PATENT SPECIFICATION

(11) **EP 3 596 228 B1**
(45) Date of publication and mention of the grant of the patent: **28.09.2022**
(21) Application number: 18720360.9
(22) Date of filing: 13.03.2018
(51) Int. Cl.: C12Q 1/68, C12Q 1/24, C12Q 1/25

(54) **METHOD FOR RAPID IDENTIFICATION OF MICROORGANISMS PRODUCING NUCLEASE ENZYMES**
VERFAHREN ZUR SCHNELLEN IDENTIFIZIERUNG VON NUKLEASE-ENZYMEN PRODUZIERENDEN MIKROORGANISMEN
PROCÉDÉ D'IDENTIFICATION RAPIDE DE MICRO-ORGANISMES PRODUISANT DES ENZYMES NUCLÉASES

(30) Priority: 14.03.2017 TR 201703901
(43) Date of publication of application: 22.01.2020
(73) Proprietor: Tubitak, 06100 Ankara (TR)
(72) Inventor: MOZIOGLU, Erkan, Atasehir, Istanbul 34684 (TR); AKYÜREK, Sema, 41470 Kocaeli (TR); GÜNDÜZ, Simay, 41470 Kocaeli (TR); AKGÖZ, Müslüm, 41470 Kocaeli (TR); GÖREN, Ahmet Ceyhan, 41400 Gebze Kocaeli (TR); KOCAGÖZ, Zühtü Tanil, Atasehir, Istanbul 34684 (TR)
(86) International application number: PCT/IB2018/051664
(87) International publication number: WO 2018/167666

(56) References cited:
- EP-A1- 2 751 567
- WO-A1-2013/033436
- WO-A1-2016/097952
- GB-A- 2 020 017
- KRISTIN EISENSCHMIDT ET AL: "A fluorimetric assay for on-line detection of DNA cleavage by restriction endonucleases", JOURNAL OF BIOTECHNOLOGY, vol. 96, no. 2, 1 June 2002 (2002-06-01), pages 185-191, XP055477485, AMSTERDAM, NL ISSN: 0168-1656, DOI: 10.1016/S0168-1656(02)00029-9
- CHAUDHURI P ET AL: "Modified methods for simple and rapid detection of bacterial deoxyribonuclease production", JOURNAL OF MICROBIOLOGICAL METHODS, ELSEVIER, AMSTERDAM, NL, vol. 16, no. 4, 1 December 1992 (1992-12-01), pages 307-311, XP023941478, ISSN: 0167-7012, DOI: 10.1016/0167-7012(92)90083-G [retrieved on 1992-12-01]
- Onur Akpinar ET AL: "Screening and regulation of alkaline extracellular protease and ribonuclease production of Yarrowia lipolytica strains isolated and identified from different cheeses in Turkey", Annals of Microbiology, 1 December 2011 (2011-12-01), pages 907-915, XP055477450, Berlin/Heidelberg DOI: 10.1007/s13213-011-0213-x Retrieved from the Internet: URL:http://www.barc.gov.in/publications/nl /2004/200410-13.pdf

## Description

### Technical Field of the Invention

The present invention relates to a method for rapid identification of microorganisms producing nuclease enzymes.

The invention more particularly relates to a method for rapid identification of microorganisms producing nuclease enzymes using the quenched DNA/RNA molecules (*fluorophore* and *quencher* labelled DNA/RNA molecules) or the gold nanoparticles accumulated on the DNA/RNA molecules.

### Prior Art

Today, the identification of nuclease enzymes (DNAse and RNAse) is typically used as in important marker.

To that end, microorganisms are grown in DNA-containing agar media, and then IN HCl is added to the grown culture and identification can be made taking into account the non-transparency of the medium. These experiments can be conducted at about 18-24 h. In order to make this duration shorter, Devran Gerçeker et al. have developed a method that differs from the conventional methods, the so-called DNAse Test Tube method. According to this, whether the organisms grown in a DNA-containing liquid medium produce nuclease enzymes is determined based on the agarose gel image subsequent to culturing. It is claimed that the presence of the bacteria can thus be rapidly identified₍₁₎. Here, however, it is inevitable that the system will not be easy to apply while working on many patient samples since gel running process, which is rather inconvenient, is added to the method as a secondary process.

In the Chinese Patent Application Numbered CN102321759 filed on 25.08.2011 within the state of the art, *'molecular beacon'* technique is employed for detecting the activity of S1 nucleases.

In the Mexican Patent Application Numbered MXPA05006448 filed on 23.12.2002 within the state of the art, *'molecular beacon'* technique is employed for detecting the activity of the nucleases cleaving the RNA.

The U.S. Patent Application Numbered US4719097 filed on 26.09.1986 within the state of the art, on the other hand, discloses the use of *resorufin* molecules while detecting the phosphatase enzyme activity.

In the German Patent Application Numbered DE19843873 filed on 25.09.1998 within the state of the art, a method has been developed in which *rhodamine* 110 molecule is used for the detection of the protease enzyme activity.

In the Patent Application Numbered WO2014207515 filed on 25.09.1998 within the state of the art, a method has been developed in which the nuclease activities can be detected eliminating the need for the quencher molecule.

The Chinese Patent Application Numbered CN101871008 filed on 31.05.2010 within the state of the art discloses the development of a chemical in gel form for use in the identification of fungal acid nucleases. The pH of this chemical, when in liquid form, is 5-6 and it comprises DNA/RNA, zinc, calcium, toluene blue and agar. A method for identifying the nucleases produced by the fungi using this chemical is disclosed.

The aforementioned documents differ from the "method for rapid identification of microorganisms producing nuclease enzymes" according to the invention in terms of content and method.

The U.S. Patent Application Numbered US2003108873 filed on 28.09.2001 within the state of the art relates to the identification of 5' nucleases and 3' exonucleases derived from microorganisms on a sequence specificity basis. This method can be employed in the identification and detection of the bacteria or viral diseases in a sample. Particularly, this method differs from the "method for rapid identification of microorganisms producing nuclease enzymes" according to the present invention in that the former is specific to sequences, and thus it is first required to detect the nucleic acid sequences, as well as in terms of the usage purpose and method.

The U.S Patent Application Numbered US2017335366 filed on 23.11.2017 within the state of the art relates to a method for the colorimetric detection of contamination with nuclease. In this document, oligonucleotide linkers of DNA/RNA which are substrate for DNAse/RNase, incubated with the sample to cause degradation of oligonucleotide linker in the case of presence of enzyme, then the colloidal gold nanoparticle (AuNP) probes are added to the test tube. Colloidal gold nanoparticles capped with citrate, functionalized with oligonucleotides 1 and 2, designated AuNP1 and AuNP2 where the oligonucleotide sequences 1 and 2 are complementary to a respective portion of the nucleotide sequence of the oligonucleotide linker. Oligonucleotide linkers of the DNA/RNA constitute the substrate for DNAase or RNAase. In the presence of an intact substrate, as a result of the hybridization event (an aggregate of AuNP1 and AuNP2 resulting from hybridization), the AuNPs become crosslinked, leading to a visible colour change from red to purple/violet/blue. In contrast, in the presence of contamination of the spesific class of nucleases that is being tested, the AuNPs do not undergo crosslinking.

The European Patent Application Numbered EP 2751567A1 filed on 09.07.2014 within the state of the art relates to a method for detecting ribonuclease (RNase)/deoxyribonuclease (DNase) activity in a test sample which could be blood serum, food, culture supernatant or washed bacteria. This document combines the test sample with a substrate thereby creates a test reaction mixture. The substrate comprises an oligonucleotide of 2-30 nucleotides in length having a cleavage domain, a fluorescence-reporter group operably linked to the one side of the oligonucleotide, and a quenching group on the other side of the nucleotide. According to the method, the test reaction mixture is incubated for a time sufficient for cleavage of the substrate by ribonuclease in the sample. The fluorescence level is detected from the test reaction mixture, which indicates that the sample contains ribonuclease activity. The method disclosed in this document requires many preparation steps for detection and the probes (substrates for detection) are not added directly to the growth medium of microorganisms. The test samples go through stages like dilution, washing, and centrifugation before mixing them with probes. Therefore, the present invention differs from this invention by eliminating the additional preparation steps such as centrifugation, culturing the bacteria, washing bacteria and dilution etc. before the assay. The procedure applied in this document is too complex, require more steps and time consuming compared to present application.

The U.S. Patent Application Numbered US2015211044 filed on 15.03.2015 within the state of the art relates to a method developed for detecting enzymes or microorganisms. In this method, after the microorganisms are concentrated first, they are placed in a solution containing a chromogenic or fluorogenic substrate. In case of the presence of enzyme, it is coupled to *chromophores* or *fluorophores.* Therefore, in said invention, the microorganisms are required to be concentrated and kept in a solution which contains substrate. Here, however, in the "method for rapid identification of microorganisms producing nuclease enzymes" according to the present invention, the fluorogenic substrate is directly added to the medium and the principle of an enzyme functioning efficiently in medium conditions is utilized. Hence, the microorganisms can be identified during the growth thereof, based on the presence of enzyme. As a result, the present invention differs from the aforementioned document in that it allows not only the determination of the number of microorganisms but also detecting their resistance against germicidal chemicals.

The lack of the use of quenched DNA/RNA molecules (*fluorophore* and *quencher* labelled DNA/RNA molecules) and the gold nanoparticles accumulated on the DNA/RNA molecules in the identification methods of the microorganisms producing nuclease has deemed it necessary to develop the method for rapid identification of microorganisms producing nuclease enzymes according to the invention.

### Objects and Summary of the Invention

The object of the present invention is to provide a method of rapidly identifying nuclease enzyme-producing microorganisms in short times expressed as minutes, depending on the bacterial density.

Another object of the present invention is to provide the method of rapid identification of microorganisms producing nuclease enzymes, by adding microorganisms to a microbiological medium having suitable physiological pH values and comprising DNA/RNA, at one terminus of which fluorogenic molecules are present while at the other terminus fluorescence quenching molecules are present, said molecules being coupled to one another.

Another object of the present invention is to provide the method for rapid identification of microorganisms producing nuclease enzymes, by seeding microorganisms in a liquid present in a tube or plate at a suitable pH value such that gold particles will remain positively charged while the DNA/RNA molecules will remain negatively charged.

And another object of the invention is to provide the method for rapid identification of microorganisms producing nuclease enzymes which allows real time monitoring of the same.

The invention relates to a rapid identification method which is used for separating the microorganisms which produce nuclease enzymes and which do not produce nuclease enzymes. Two different types of content can be used in the method. And these are:
o Seeding the microorganisms to a liquid or solid microbiological medium containing quenched DNA/RNA molecules (*fluorophore* and *quencher* labelled DNA/RNA molecules). The organisms producing nuclease enzymes in this content are detected by means of the fluorescence likely to be observed in the medium.
o Seeding the microorganisms to the liquids containing gold nanoparticles and DNA/RNA molecules. In this content, on the other hand, the organisms producing nuclease enzyme are detected by the discoloration likely to be observed in the liquid.

### Detailed Description of the Invention

The method for rapid identification of microorganisms producing nuclease enzymes according to the invention is illustrated in the drawings, in which:
**Fig. 1** is the reproduction-dependent change curve of *S. pyogenes,* which is an organism that produces nuclease enzymes.
**Fig. 2** is the fluorescence change curve of *S. aureus,* which is an organism that produces nuclease enzymes, dependent on the reproduction of other microorganisms not producing nuclease enzymes.
**Fig. 3** is the fluorescence change curve of *S. aureus,* which is an organism that produces nuclease enzymes, dependent on reproduction at different initial concentrations.

The invention is a method for rapid identification of microorganisms producing nuclease enzymes, comprising the steps of:
- Coupling to one terminus of the DNA/RNA molecules the fluorogenic/fluorescence molecules (*fluorophore*)*,* and to the other terminus the fluorescence quenching (*quencher*) molecules,
- Preparing a solution comprising such DNA/RNA molecules and placing the former in the medium,
- Adding the microorganisms to be identified to the medium, and
- Monitoring the fluorescence signal in the medium by means of an optical apparatus.

The invention is a method for rapid identification of microorganisms producing nuclease enzymes, comprising the steps of:
- Preparing a solution consisting of positively charged (positive) gold nanoparticles and negatively charged (negative) DNA/RNA molecules,
- Adding the microorganisms to be identified to the thus prepared solution, and
- Monitoring the discoloration likely to occur in the solution.

The invention is a method for rapid identification of microorganisms producing nuclease enzymes, wherein a kit comprising a medium having the suitable physiological pH value and in which quenched DNA/RNA (*fluorophore* and *quencher* labelled DNA/RNA) molecules are present, is used.

The invention is a method for rapid identification of microorganisms producing nuclease enzymes, wherein a kit comprising a medium having the suitable physiological pH value and in which quenched DNA/RNA (*fluorophore* and *quencher* labelled DNA/RNA) molecules are present; suitable tubes and/or plates for fluorescence studies; a shaking or non-shaking incubator to be used in the growth of microorganisms; an optical apparatus for detecting and evaluating the fluorescence signals likely to occur after seeding the microorganisms; and a device with software for the assessment of the signals are used.

The invention is a method for rapid identification of microorganisms producing nuclease enzymes, wherein a kit which consists of positively charged gold nanoparticles and negatively charged DNA/RNA molecules and which comprises a solution having the suitable pH value is used.

The invention is a method for rapid identification of microorganisms producing nuclease enzymes, wherein a kit which consists of positively charged gold nanoparticles and negatively charged DNA/RNA molecules and which comprises a solution having the suitable pH value; tubes and/or plates suitable for discoloration; an optical apparatus for sensing and evaluating the absorption signals associated with the discoloration to occur after seeding the microorganisms; and a device with software used for the assessment of the signals are used.

In an embodiment of the method according to the invention, the fluorescence signal is low in case no microorganism is seeded to a microbiological medium with suitable physiological pH values which comprises the DNA/RNA molecules which at one terminus is labelled with the fluorophore molecules, and with the quencher molecules at the other terminus.

For a rapid identification of the microorganisms producing nuclease enzymes, such microorganisms are seeded to a microbiological medium having suitable physiological pH values and comprising the DNA/RNA molecules which are labelled with fluorophore molecules at one terminus, and with quencher molecules at the other. In case the microorganisms produce nuclease enzyme, the nuclease enzymes of such microorganisms cleave the DNA/RNA molecules. Hence, the quencher molecules and the fluorophore molecules are distanced from one another. An increase in the fluorescence signals is thus observed. Since this change in signal is directly associated with the presence of the nuclease enzymes, and thus of the microorganisms that are capable of producing nuclease enzymes, the identification of such microorganisms dependent on the enzyme production and/or bacterial density can be made in a short time, e.g. 10 minutes to 8 hours.

If the microorganisms do not produce nuclease enzyme, no change in the fluorescence signal is observed due to the absence of cleavage in DNA/RNA molecules.

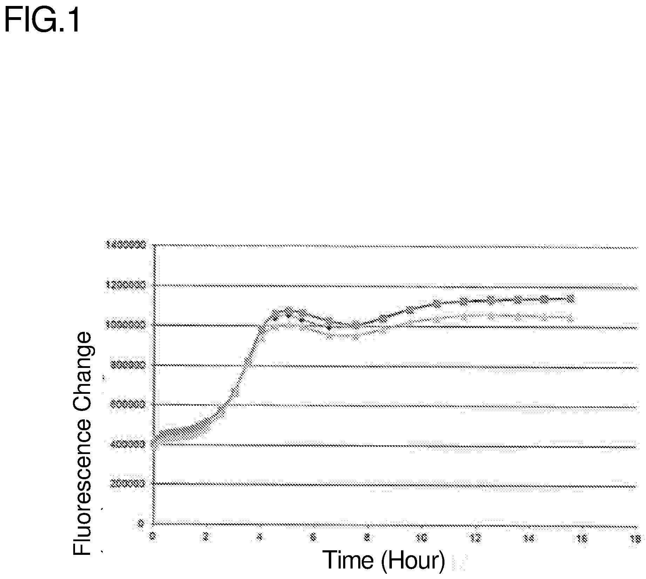

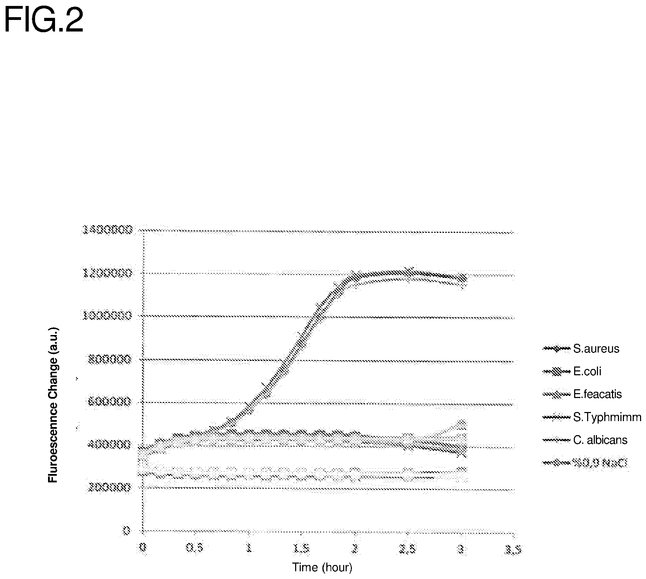

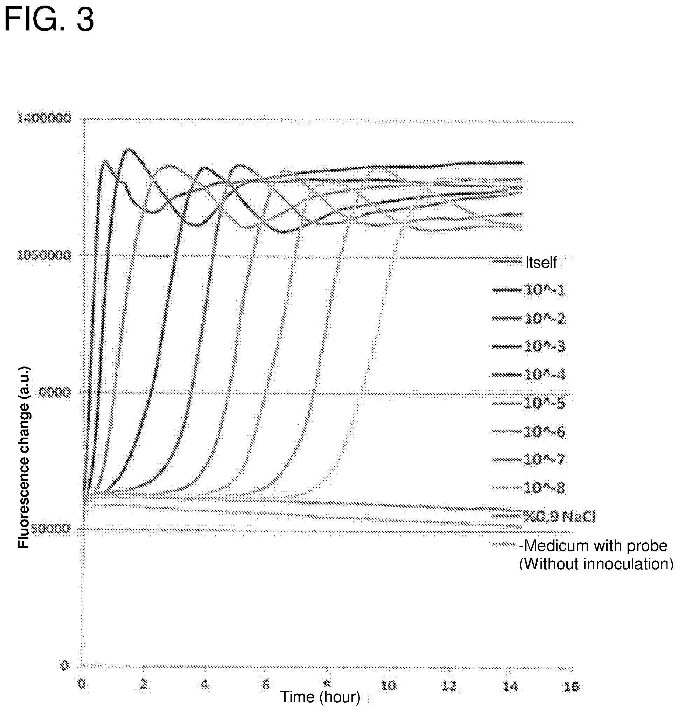

This fluorescence-based method is not only rapid, but also it allows real time monitoring, which, in turn, makes it possible to perform qualitative (present/absent) identification of the microorganisms producing nuclease enzyme as well as quantitative (bacteria count) identification thereof (Figs. 1, 2, and 3).

Again, this fluorescence-based method, thanks to the ability of determining the bacteria count, may as well be used in the experiments in which the lowest germicidal (*antibiotic*/*antifungal*) concentration of the microorganisms producing nuclease enzymes is detected.

In the method according to the invention, monitoring of the fluorescence signals in the solution is conducted by means of an optical apparatus. The signals are converted and analyzed by the software.

In another embodiment of the method according to the invention, the gold nanoparticles and DNA/RNA molecules are present in a positively charged and negatively charged state, respectively, in the liquid which comprises gold nanoparticles and DNA/RNA molecules and which has a certain pH value. In this case, charge interaction takes place between the negatively charged DNA/RNA molecules and positively charged gold nanoparticles. As a consequence of such interaction, gold nanoparticles accumulate on the DNA/RNA molecules, making the color of the solution change from red to blue.

Because the solution which turned blue in the presence of the DNA/RNA molecules will be cleaved by the nuclease enzymes of the DNA/RNA molecules in the presence of the microorganisms which are placed therein and which are capable of producing nuclease enzymes, the accumulated gold nanoparticles are dispersed in the solution again, making the color of the solution red again. This visible change can be utilized in the rapid identification of the microorganisms producing nuclease enzymes.

The discoloration does not require, apart from the gold nanoparticles, any other chromogenic chemical, e.g. HMRZ-86 ((7R)-7-[2- (aminothiazol-4-1)-(z)-2-(1-carboxy-1-methylethoxyimino)acetamido]-3-(2,4-dinitrostyryl)-3-cephem-4-carboxylic acid trifluoroacetate, E-isomer).

### REFERENCES

(1) Gerceker D, Karasartova D, Elyürek E, Barkar S, Kiyan M, Ozsan TM, Calgin MK, Sahin F. 2009. A new, simple, rapid test for detection of DNase activity of microorganisms: DNase Tube test. J Gen Appl Microbiol. 55 (4):291-4.

## Claims

1. A method for real-time monitoring and rapid identification of microorganisms producing nuclease enzymes, **characterized in that** it comprises the steps of:
- coupling to one terminus of the DNA/RNA molecules the fluorogenic molecules (*fluorophore*)*,* and to the other terminus the fluorescence quenching (*quencher*) molecules,
- preparing a solution comprising said DNA/RNA molecules with fluorogenic and quenching molecules and placing the solution into the growth medium,
- seeding the microorganisms to be identified to the growth medium containing the solution comprising said DNA/RNA molecules with fluorogenic and quenching molecules, and
- monitoring the fluorescence signal in the growth medium during the growth of microorganism by means of an optical apparatus.

2. A method for rapid identification of microorganisms producing nuclease enzymes, **characterized that** it comprises the steps of:
- preparing a solution consisting of positively charged gold nanoparticles and negatively charged DNA/RNA molecules,
- adding the microorganisms to be identified to the solution, and
- monitoring the discoloration in the solution.

3. A method according to Claim 1 **characterized in that** it uses a kit comprising a medium containing a solution having a suitable physiological pH value.

4. A method according to Claim 3, **characterized in that** the used kit further comprises; suitable tubes and/or plates for fluorescence studies; a shaking or non-shaking incubator to be used in the growth of microorganisms; an optical apparatus for detecting and evaluating the fluorescence signals likely to occur after seeding the microorganisms; and a device with software for the assessment of the signals.

5. A method for rapid identification of microorganisms producing nuclease enzymes according to Claim 2, **characterized in that** it uses a kit which consists of positively charged gold nanoparticles and negatively charged DNA/RNA molecules and which comprises a solution having the suitable pH value.

6. A method for rapid identification of microorganisms producing nuclease enzymes according to Claim 5, **characterized in that** the used kit further comprises tubes and/or plates suitable for discoloration; an optical apparatus for sensing and evaluating the absorption signals associated with the discoloration to occur after seeding the microorganisms; and a device with software for the assessment of the signals.

## Patentansprüche

1. Verfahren zur Echtzeitüberwachung und schnellen Identifizierung von Mikroorganismen, die Nuklease-Enzyme produzieren, **dadurch gekennzeichnet, dass** es die folgenden Schritte umfasst:
- Kopplung der fluorogenen Moleküle (*Fluorophore*) an einen Endpunkt der DNA/RNA-Moleküle und der fluoreszenzlöschenden Moleküle (*Quencher*) an den anderen Endpunkt,
- Herstellung einer Lösung, die die DNA/RNA-Moleküle mit fluorogenen Quenchmolekülen enthält, und Einbringen der Lösung in das Wachstumsmedium,
- Aussäen der zu identifizierenden Mikroorganismen in das Wachstumsmedium, das die Lösung enthält, die die DNA/RNA-Moleküle mit fluorogenen Quenchmolekülen enthält, und
- Überwachung des Fluoreszenzsignals im Wachstumsmedium während des Wachstums der Mikroorganismen mit Hilfe eines optischen Geräts.

2. Verfahren zur schnellen Identifizierung von Mikroorganismen, die Nuklease-Enzyme produzieren, **dadurch gekennzeichnet, dass** es die folgenden Schritte umfasst:
- Herstellung einer Lösung, die aus positiv geladenen Gold-Nanopartikeln und negativ geladenen DNA/RNA-Molekülen besteht,
- Zugabe der zu identifizierenden Mikroorganismen zu der Lösung, und
- Überwachung der Verfärbung der Lösung.

3. Verfahren nach Anspruch 1, **dadurch gekennzeichnet, dass** es ein Kit verwendet, das ein Medium umfasst, das eine Lösung mit einem geeigneten physiologischen pH-Wert enthält.

4. Verfahren nach Anspruch 3, **dadurch gekennzeichnet, dass** der verwendete Kit weiterhin geeignete Röhrchen und/oder Platten für Fluoreszenzstudien, einen Schüttel- oder Nicht-Schüttel-Inkubator, der für das Wachstum von Mikroorganismen zu verwenden ist, ein optisches Gerät zum Nachweis und zur Auswertung der Fluoreszenzsignale, die nach dem Aussäen der Mikroorganismen wahrscheinlich auftreten, und eine Vorrichtung mit Software zur Auswertung der Signale umfasst.

5. Verfahren zur schnellen Identifizierung von Mikroorganismen, die Nuklease-Enzyme produzieren, nach Anspruch 2, **dadurch gekennzeichnet, dass** es ein Kit verwendet, das aus positiv geladenen Gold-Nanopartikeln und negativ geladenen DNA/RNA-Molekülen besteht und eine Lösung mit dem geeigneten pH-Wert enthält.

6. Verfahren zur schnellen Identifizierung von Mikroorganismen, die Nuklease-Enzyme produzieren, nach Anspruch 5, **dadurch gekennzeichnet, dass** der verwendete Kit weiterhin die für die Verfärbung geeigneten Röhrchen und/oder Platten, ein optisches Gerät zum Erfassen und Auswerten der mit der Verfärbung verbundenen Absorptionssignale, die nach dem Aussäen der Mikroorganismen auftreten sollen, und eine Vorrichtung mit Software für die Auswertung der Signale umfasst.

## Revendications

1. Procédé de surveillance en temps réel et d'identification rapide de microorganismes produisant des enzymes nucléases, **caractérisé en ce qu'il** comprend les étapes de :
- couplage des molécules fluorogènes (*fluorophore*) à une terminaison des molécules d'ADN/ARN et des molécules d'extinction de fluorescence (*extincteur*) à l'autre terminaison,
- préparation d'une solution comprenant lesdites molécules d'ADN/ARN avec des molécules fluorogènes et d'extinction et placement de la solution dans le milieu de croissance,
- d'ensemencement des microorganismes à identifier dans le milieu de croissance contenant la solution comprenant lesdites molécules d'ADN/ARN avec des molécules fluorogènes et d'extinction, et
- surveillance du signal de fluorescence dans le milieu de croissance pendant la croissance du microorganisme au moyen d'un appareil optique.

2. Procédé d'identification rapide de microorganismes produisant des enzymes nucléases, **caractérisé en ce qu'il** comprend les étapes de :
- préparation d'une solution constituée de nanoparticules d'or chargées positivement et de molécules d'ADN/ARN chargées négativement,
- ajout des microorganismes à identifier à la solution, et
- surveillance de la décoloration dans la solution.

3. Procédé selon la Revendication 1, **caractérisé en ce qu'il** utilise un kit comprenant un milieu contenant une solution ayant une valeur de pH physiologique appropriée.

4. Procédé selon la Revendication 3, **caractérisé en ce que** le kit utilisé comprend en outre ; des tubes et/ou plaques appropriés pour les études de fluorescence ; un incubateur à agitation ou sans agitation à utiliser dans la croissance de microorganismes ; un appareil optique de détection et d'évaluation des signaux de fluorescence susceptibles de se produire après l'ensemencement des microorganismes ; et un dispositif avec un logiciel pour l'évaluation des signaux.

5. Procédé d'identification rapide de microorganismes produisant des enzymes nucléases selon la Revendication 2, **caractérisé en ce qu'il** utilise un kit qui est constitué de nanoparticules d'or chargées positivement et de molécules d'ADN/ARN chargées négativement et qui comprend une solution ayant la valeur de pH appropriée.

6. Procédé d'identification rapide de microorganismes produisant des enzymes nucléases selon la Revendication 5, **caractérisé en ce que** le kit utilisé comprend en outre des tubes et/ou plaques appropriés pour la décoloration ; un appareil optique pour détecter et évaluer les signaux d'absorption associés à la décoloration devant se produire après l'ensemencement des microorganismes ; et un dispositif avec un logiciel pour l'évaluation des signaux.
